# EUROPEAN PATENT SPECIFICATION

(11) **EP 2 022 492 B1**
(45) Date of publication and mention of the grant of the patent: **16.03.2016**
(21) Application number: 08010499.5
(22) Date of filing: 10.06.2008
(51) Int. Cl.: A61K 31/16, A61K 31/165, A61K 31/19, A61K 31/192, A61K 31/203, A61K 31/245, A61K 31/353, A61K 31/455, A61K 38/12, A61P 35/00

(54) **Anti-cancer pharmaceutical composition**
Pharmazeutische Antikrebszusammensetzung
Composition pharmaceutique anticancer

(30) Priority: 10.08.2007 IT GE20070079
(43) Date of publication of application: 11.02.2009
(73) Proprietor: Pianezza, Maurizio, 16146 Genova (GE) (IT)
(72) Inventor: Pianezza, Maurizio, 16146 Genova (GE) (IT)
(74) Representative: Forattini, Amelia

(56) References cited:
- US-A1- 2002 183 388
- US-A1- 2005 288 310
- STRESEMANN C ET AL: "Functional diversity of DNA methyltransferase inhibitors in human cancer cell lines" CANCER RESEARCH 20060301 US, vol. 66, no. 5, 1 March 2006 (2006-03-01), pages 2794-2800, XP002491322 ISSN: 0008-5472
- GHOSHAL K ET AL: "INHIBITORS OF HISTONE DEACETYLASE AND DNA METHYLTRANSFERASE SYNERGISTICALLY ACTIVATE THE METHYLATED METALLOTHIONEIN I PROMOTER BY ACTIVATING THE TRANSCRIPTION FACTOR MTF-1 AND FORMING AN OPEN CHROMATIN STRUCTURE" MOLECULAR AND CELLULAR BIOLOGY, AMERICAN SOCIETY FOR MICROBIOLOGY, WASHINGTON, US, vol. 22, no. 23, 1 December 2002 (2002-12-01), pages 8302-8319, XP008039464 ISSN: 0270-7306
- PATRA S K ET AL: "Methyl-CpG-DNA binding proteins in human prostate cancer: Expression of CXXC sequence containing MBD1 and repression of MBD2 and MeCP2" BIOCHEMICAL AND BIOPHYSICAL RESEARCH COMMUNICATIONS 20030321 US, vol. 302, no. 4, 21 March 2003 (2003-03-21), pages 759-766, XP002491323 ISSN: 0006-291X
- CHINNAIYAN P ET AL: "Enhancing the antitumor activity of ErbB blockade with histone deacetylase (HDAC) inhibition" INTERNATIONAL JOURNAL OF CANCER 20060215 US, vol. 118, no. 4, 15 February 2006 (2006-02-15), pages 1041-1050, XP002491324 ISSN: 0020-7136 1097-0215

## Description

The present invention relates to a multicomponent pharmaceutical composition, particularly for treating cancerous disease and a form of cancer such as prostate cancer.

In eukaryotic cells, DNA is stored in a higher-order structure known as chromatin. This structure assumes the form of a condensed and very dense packet of DNA in which there are regular and repeated units known as nucleosomes.

Chromatin is constituted predominantly by nucleic acids (DNA and RNA), characterized by a negative charge, and by two types of protein: histones, which have a positive charge, and nonhistone chromosomal proteins, which also have a positive charge when they are in an environment with neutral pH.

Structurally, chromatin assumes two different forms: the first one, known as heterochromatin, is particularly compact and therefore inactive in terms of gene transcription. The second form is known as euchromatin and is characterized by a less compact structure which is accessible to cellular transcription mechanisms and is therefore involved in gene expression and transcription processes.

It is known that the degeneration of gene transcription control mechanisms and of epigenetic mechanisms is closely tied to tumor-type diseases.

Control over gene transcription is performed also by means of changes at the level of chromatin structure: these changes are mediated by chemical modifications on the histones, which occur predominantly at sites in the aminoterminal portion (N-terminal) portion, where numerous residues of lysine are located which are fundamental to give a positive charge to the histones. The modification mechanisms are within the group of processes for methylation, acetylation/deacetylation, phosphorylation, ubiquitination, sumoylation and ADP-ribosylation (Marks et al., 2000-2003-2004). Among these, the most widely studied processes are those which involve methylation and acetylation/deacetylation.

Modification at the histone level for acetylation/deacetylation is regulated by a series of various histone-acetyltransferase (HAT) enzymes and histone-deacetylase (HDAC) enzymes. HAT enzymes bind acetyl groups on lysine residues of histones, masking the positive charges, and also act on other proteins and enzymes, such as for example some components of the transcription system, favoring gene transcription. HDAC enzymes instead return the positive charge to the histones, removing the acetyl groups from them, inducing compaction of euchromatin with an unfavorable action on gene transcription.

The effect is a blockage of gene transcription, because the positive charge of the histones can interact with the negative charge of the DNA, leading to the assumption of a configuration which is unfavorable to transcription.

Current knowledge of HAT/HDAC imbalances allows to say that this can constitute a means for interrupting transcription regulation and, although direct alterations in HDAC genes in cancer have not been demonstrated, the association of HDAC with various oncogens and tumor suppressors is now well established, thus demonstrating the importance of epigenetic mechanisms.

Normally, the state of acetylation is tightly controlled by the antagonistic activity of HAT and HDAC, and the effect of this balance affects histones, the gene transcription system, and other important biochemical targets, such as the p53 tumor suppressor (Grozinger and Schreiber 2002).

Known HAT enzymes fall within various groups, such as the Gen 5 linker N-acetyltransferase (GNAT) family, the CREB protein linker (p300/CBP) family, and the monocytic leukemia Zn-protein (MYST) group. It is known that the genes related to these enzymes are hyperexpressed in many blood tumors and solid tumors, thus stressing the importance of the acetylation system within the cellular cycle. Disorganized hyperacetylation activates the promoters that are normally repressed, leading to inappropriate protein synthesis and to an inhibitory action on gene expression for a given phenotype, thus acting as a disorder-provoking factor.

HDAC enzymes are in turn divided into three classes, based on structure homology. Class 1 and 2 HDACs are characterized by the presence of a zinc atom within the active site and respond to the action of specific inhibitors. Class 3 HDACs (sirtuins) are evolutionarily distinct from the preceding ones and their inhibition is linked to the presence of nicotinamide.

It is known that various HDAC enzymes are involved in cell differentiation processes and indeed some forms of tumor exhibit a high expression of these enzymes. Furthermore, the involvement of class 3 HDACs in the modulation of p53-mediated apoptotic response indicates that these enzymes can be the target of anti-cancer pharmacological therapies.

Class 1 HDAC (Rpd3) analogs have isoforms such as 1, 2, 3 and 8, which are expressed preponderantly in all tissues. Class 2 HDAC (Hda) homologues have isoforms such as 4, 5, 6, 7, 9a, 9b, and 10, which are expressed in a more limited number of tissues.

Among these HDAC isoforms, 4, 8 and 9 are demonstrably expressed in cancer. However, it should be assumed that other isoforms are present in cancer cells and in any case belong to both HDAC classes. For class 3 HDAC homologues, which are ubiquitously present in tumor tissues, their dependency on nicotinamide and on their ability to induce apoptosis, for example by means of the block induced on SIR2-alpha, leading to increased p53 levels or limiting forkhead proteins for SIR1, inducing apoptosis, applies as already mentioned.

Accordingly, HDAC enzyme inhibitors are effective on a wide range of neoplastic cells, although they remain relatively non-toxic for normal cells (Rosato-Grant 2003; Zhu et al. 2001).

The administration of compounds which act on the acetylation/deacetylation system is, therefore, among the cures suitable for treating potentially all forms of cancer, restoring compromised transcription mechanisms and inducing apoptosis in tumor cells.

However, due to the complex nature of the biochemical mechanisms involved or altered by tumor forms, the use of individual compounds which act on a single biochemical pathway is not ideal for effective treatment in patients affected by a form of cancer. US2002183388 discloses a pharmaceutical composition comprising at least one histone deacetylase (HDAC) inhibitor, such as sodium phenylbutyrate or valproic acid, and a retinoid, such as all-trans retinoic acid (ATRA) for the treatment of solid tumors.

US2005288310 discloses nicotinamide as PARP-1 (poly (ADP-ribose)polymerase-1) inhibitor in combination with naphthalimide for treating prostate cancer.

There is, therefore, the need to provide new pharmaceutical formulations which can act synergistically on multiple biochemical mechanisms simultaneously, so as to allow a more effective anti-cancer treatment. Indeed, one can begin to activate the transcription repressed in various tumor phenotypes from an induced hyperacetylation effect.

The aim of the present invention is to provide a pharmaceutical composition which acts on the enzymes of the acetylation/deacetylation system synergistically.

Within the scope of this aim, an object of the invention is to provide a composition whose synergistic action on the enzymes of the acetylation/deacetylation system is accompanied by an action on the enzymes that regulate methylation.

A further object of the invention is to provide a composition which can augment the effect of anti-cancer therapies, particularly in prostate carcinoma.

This aim and these and other objects, which will become better apparent hereinafter, are achieved by a pharmaceutical composition comprising ingredients, where:
- a first active ingredient is sodium phenylbutyrate in combination with valproic acid;
- a second active ingredient is nicotinamide; and
- a third active ingredient is all-trans retinoic acid (ATRA).

The aim and objects of the invention are also achieved by a composition as described here for the treatment of a form of cancer, particularly by oral administration of the composition.

According to the invention, the pharmaceutical composition of claim 1 has a synergistic effect, acting on class 1, 2 and 3 HDAC enzymes and on methylation mechanisms, so as to cause histone hyperacetylation and demethylation phenomena.

The first active ingredient of the composition according to the invention is constituted by two compounds capable of inhibiting histone deacetylation by acting on HDAC enzymes.

This inventor has in fact discovered that these compounds, if taken simultaneously, are capable of rebalancing synergistically the antagonistic action of HAT/HDAC enzymes. It is believed that the action of these molecules is linked to irreversibility and to the capacity to act at various levels with the catalytic pocket of HDAC enzymes, although due to the limited length of their hydrocarbon chains valproic acid and sodium phenylbutyrate interact with limited affinity with the enzyme pocket.

Furthermore, phenylbutyrate induces expression of the CDKNA-1 gene, which codes for a cyclin-dependent protein kinase. This kinase in turn is capable of inducing apoptosis in addition to G0-G1 block of the cell cycle by inhibiting the P21 Waf protein. Furthermore, phenylbutyrate is capable of interfering with the cell cycle mechanism by means of a coordinated inactivation of cytoprotection of the RAF/MEK/ERK system, the pathway through which ROS-dependent activation of JNK occurs.

As regards valproic acid, this molecule acts by inducing selective proteasomal degradation of class 2 HDACs, inducing apoptosis. Valproic acid is also characterized by an antiangiogenic activity.

The second active ingredient of the composition according to the invention is nicotinamide, whose action affects class 3 HDAC enzymes. Nicotinic acid amides in fact have an inhibitory effect on NAD-dependent p53 deacetylation performed by a class 3 HDAC enzyme. The direct effect consists in the permanence of p53 in a state of acetylation, by means of which it facilitates the activation of apoptosis mechanisms. The action of nicotinic acid amides can be based on an acetyl-ADP-ribosylation mechanism, on modulation of intracellular calcium control by means of calmodulin, or by means of channel receptors known as ryanodine receptors.

The third active ingredient of the composition according to an invention is all-trans retinoic acid (ATRA).

Advantageously, the compound of the third active ingredient has an action on demethylation processes which is synergistic with the inhibition of HDAC enzymes.

In particular, retinoids, such as for example ATRA, act according to two mechanisms of action, the first one at the apoptosis level and the second one at the level of methylation mechanisms with a pro-differentiation effect. ATRA is in fact capable of acting at the level of the balance between the bc12 and bax factors, facilitating the latter and thus initiating cell apoptosis. The second action of ATRA is linked to its interaction with RAR (Retinoic Acid Receptor) and XRX (Rexinoid Acid Receptor) receptors; in particular, RAR-beta is known as a tumor suppressor whose function is compromised in various forms of cancer, including prostate cancer, lung cancer, breast cancer, esophageal cancer and squamous tumors of the head and neck. ATRA activates the RAR and XRX receptors, dimerizing them; these in turn cause inhibition of cell development and apoptosis induction. Furthermore, ATRA, by interfering with the methylation mechanism of CpG sites, contributes both to reactivation of key enzymes in protection against tumors and to the activation of enzymes which inhibit transcription, such as HDAC enzymes. These mechanisms are a further indicator of the synergistic activity performed by retinoids in combination with the other molecules of the composition, whose activity occurs at the level of HAT/HDAC enzymes.

In another embodiment, the composition according to the invention also includes an active ingredient capable of acting on DNA methyltransferase (DNMT) mechanisms.

Preferably, this active ingredient is selected from the group constituted by procaine or a pharmaceutically acceptable salt thereof in combination with benzoic acid, potassium metabisulfite and disodium phosphate, and an epigallocatenin.

In particular, the procaine salt may be procaine hydrochloride. Furthermore, procaine and its salts require combination with benzoic acid, potassium metabisulfite and disodium phosphate, because these substances avoid the rapid hydrolysis of procaine, a phenomenon which would not allow a blood dosage which would be significant for therapeutic purposes.

The role of DNMT enzymes consists in methylation of CpG sites, so that the presence of active ingredients, such as for example procaine hydrochloride, which act on the activity of DNMTs, has an additional synergistic effect on the anti-cancer action of the composition according to the invention, inhibiting the demethylation of protein structures.

In particular, procaine, by not being a molecule of the nucleoside family, applies its effect without damaging normal cells, because it does not need to be incorporated in the structure of DNA in order to act.

In an embodiment of the composition according to the invention, the butyric acid derivative sodium phenylbutyrate is in a quantity between 125 mg and 20 g by weight; the carboxylic acid having 5 to 10 carbon atoms valproic acid is in a quantity between 50 and 1.6 g by weight; the nicotinic acid amide nicotinamide is in a quantity between 250 and 1,5 g; and the retinoid ATRA is in a quantity between 2.5 and 90 mg by weight.

In another embodiment, the composition may also include procaine or a pharmaceutically acceptable salt thereof in a quantity between 20 and 600 mg by weight in combination with benzoic acid, potassium metabisulfite, and disodium potassium phosphate.

A composition including 100 mg of procaine or a pharmaceutically acceptable salt thereof, is preferably in combination with benzoic acid in a quantity between 6 and 8 mg by weight, preferably 7 mg, potassium metabisulfite in a quantity between 3 and 5 mg by weight, preferably 4 mg, and disodium potassium phosphate in a quantity between 0.1 and 0.5 mg by weight, preferably 1 mg.

A composition including 300 mg of procaine or a pharmaceutically acceptable salt thereof, is preferably in combination with benzoic acid in a quantity between 18 and 24 mg by weight, preferably 21 mg.

A composition including 600 mg of procaine or a pharmaceutically acceptable salt thereof, is preferably in combination with benzoic acid in a quantity between 36 and 48 mg by weight, preferably 42 mg.

Furthermore, the composition according to the invention may also include any pharmaceutical excipient or combinations thereof normally used in the preparation of pharmaceutical compositions.

The present invention relates also to a form of dosage which includes the composition described here. Preferably, this form of dosage includes:
- a first gastroresistant tablet including sodium phenylbutyrate and valproic acid;
- a second tablet including nicotinamide, procaine hydrochloride in combination with benzoic acid, potassium metabisulfite and disodium potassium phosphate; and
- a single soft capsule including the all-trans retinoic acid (ATRA), particularly wherein said first gastroresistant tablet, said second gastroresistant tablet and said fist single soft capsule are incorporated into a hard capsule.

Preferably, in the form of dosage according to the invention:
- the butyric acid derivative is sodium phenylbutyrate in a quantity of 250 mg;
- the carboxylic acid having 5 to 10 carbon atoms is valproic acid in a quantity of 150 mg;
- the nicotinic acid amide is nicotinamide in a quantity of 250 mg;
- the retinoid is ATRA in a quantity of 5 mg.

Furthermore, in the form of dosage it is possible to provide for the optional presence of a salt of procaine, particularly procaine hydrochloride, in a quantity of 50 mg in combination with benzoic acid in a quantity of 3.5 mg by weight, potassium metabisulfite in a quantity of 2 mg by weight, and sodium potassium sulfate in a quantity of 0.5 mg by weight.

Generally, the soft capsule and the two gastroresistant tablets must be kept separate, but they can be collected in a single blister.

In an alternative form of dosage, the soft capsule and the two gastroresistant tablets can be further incorporated into a single hard capsule or cap.

The term "tablet" is understood to reference single-dose pharmaceutical forms prepared by compacting solid substances in powder or granular form with the aid of suitable excipients; these tablets can be simple or gastroresistant, i.e., coated with protective films which dissolve only in the intestine. Substances which can be attacked either by enzymes or by the intestinal environment are used. Generally, these substances contain ester groups which are hydrolyzed in the intestine but not in the stomach; for example, phenyl salicylate (salol), which is an ester which can be hydrolyzed only at an alkaline or neutral pH, or some waxes, again of an ester type, are used. The soft capsule is a single-dose pharmaceutical form for oral use, inside which it is possible to introduce granulates, powders or oily mixes. The capsules are essentially constituted by gelatin, by a plasticizing agent (glycerin, sorbitol, simple syrup, starch), preservatives (for example small amounts of sulfur dioxide to prevent the growth of molds and bacteria on the gelatin, which is an excellent culture medium), and natural and opacifying colors (in the absence of a color, generally titanium dioxide, the capsule is transparent). Hard capsules (also known as caps) are prepared empty and can provide for the assembly of different active ingredients in different forms.

The composition according to the invention may have any other suitable form of dosage, in particular suitable for taking it orally.

In another aspect, the present invention provides a pharmaceutical composition as described here for treating a form of cancer, particularly by administering the composition orally.

Preferably, the composition for treatment of a form of cancer can be in one of the forms of dosage described here.

The retinoid is contained in a single soft capsule, the butyric acid derivative is in the form of a gastroresistant tablet, and the carboxylic acid having 5 to 10 carbon atoms and the nicotinic acid amide are in a second gastroresistant tablet. When present, the procaine or salt thereof in combination with benzoic acid, potassium metabisulfite and disodium phosphate is also contained in the second gastroresistant capsule.

In a preferred form, the single soft capsule, the first gastroresistant tablet and the second gastroresistant tablet are incorporated into a single hard capsule. This facilitates taking by a patient, since all the components are enclosed in a single assembly.

Preferably, the composition for treating a form of cancer described here is administered orally with a dosage of 2 to 6 dosage forms/day.

Of course, the dosage intervals can be modulated and established as a function of the form of cancer being treated and of the response of the patient to the drug.

The composition described here in particular is advantageous for treating a form of cancer, wherein the form of cancer is prostate cancer.

Advantageously, the composition described here can be administered together with an anti-cancer therapy. The anti-cancer therapy can be selected from the group constituted by chemotherapy and treatment with a tyrosine kinase blocking drug.

In practice it has been found that the composition according to the invention fully achieves the intended aim. The combination of the active ingredients indicated here has a synergistic action on acetylation/deacetylation mechanisms and on methylation control.

Furthermore, it has been found that the composition described here can be used in the treatment of a form of cancer, such as for example prostate cancer, since the action on acetylation/deacetylation mechanisms and methylation control performed by the components of the composition activate a neoplastic cell differentiation and apoptosis mechanism.

It has been found that the composition described here can be used for the treatment of a form of cancer and allows combination with other therapies normally used in cancer treatment.

## Claims

1. A pharmaceutical composition comprising active ingredients, wherein:
- a first active ingredient is sodium phenylbutyrate in combination with valproic acid;
- a second active ingredient is nicotinamide; and
- a third active ingredient is all-trans retinoic acid (ATRA),
and optionally one or more pharmaceutically acceptable excipients.

2. The composition according to claim 1, comprising an active ingredient capable of acting on DNA methyltransferase (DNMT) mechanisms selected from the group consisting of an epigallocatechin and procaine or an acceptable salt thereof in combination with benzoic acid, potassium metabisulfite and disodium phosphate.

3. The composition according to claim 1, comprising sodium phenylbutyrate in a quantity between 125 mg and 20 g; valproic acid in a quantity between 50 mg and 1.6 g; nicotinamide in a quantity between 250 mg and 1.5 g; and ATRA in a quantity between 2.5 and 90 mg.

4. The composition according to claim 3, furthermore comprising procaine or an acceptable salt thereof in a quantity of 50 to 600 mg in combination with benzoic acid in a quantity from 6 to 8 mg per 100 mg of procaine, potassium metabisulfite in a quantity from 3 to 5 mg per 100 mg of procaine, and disodium potassium phosphate in a quantity from 0.1 to 1.5 mg per 100 mg of procaine.

5. A dosage form, particularly for oral administration, comprising a composition according to claim 1, said dosage form comprising:
- a first gastroresistant tablet including sodium phenylbutyrate and valproic acid;
- a second tablet including nicotinamide and procaine hydrochloride in combination with benzoic acid, potassium metabisulfite and disodium potassium phosphate; and
- a single soft capsule including the all-trans retinoic acid (ATRA),
wherein optionally said first gastroresistant tablet, said second gastroresistant tablet and said first single soft capsule are incorporated into a hard capsule.

6. The dosage form according to claim 5, comprising:
- sodium phenylbutyrate in a quantity of 250 mg;
- valproic acid in a quantity of 150 mg;
- nicotinamide in a quantity of 250 mg;
- all-trans retinoic acid (ATRA) in a quantity of 5 mg.

7. The dosage form according to one or more of claims 5-6, comprising procaine hydrochloride in a quantity between 20 and 600 mg and wherein each 100 mg of procaine hydrochloride is in combination with benzoic acid in a quantity between 6 and 8 mg, potassium metabisulfite in a quantity between 3 and 5 mg, and disodium potassium phosphate in a quantity between 0.1 and 0.5 mg.

8. The dosage form according to claim 7 wherein each 100 mg of procaine hydrochloride is in combination with benzoic acid in a quantity of 7 mg, potassium metabisulfite in a quantity of 4 mg, and disodium potassium phosphate in a quantity of 1 mg.

9. The composition according to one or more of claims 1 to 4 for use in the treatment of a form of cancer.

10. The composition for use according to claim 9, wherein the treatment is by oral administration.

11. The composition for use according to claim 9, wherein the cancer is prostate cancer.

12. The composition for use according to claim 9, wherein said composition is in a dosage form according to one or more of claims 5-6.

13. The composition for use according to claim 12, wherein the composition is administered orally.

14. The composition for use according to claim 13, wherein the composition is administered with a dosage of 2 to 6 of said dosage form/day.

15. The composition for use according to one of claims 13 and 14, wherein said composition is administered in combination with an anti-cancer therapy.

16. The composition for use according to claim 15, wherein the anti-cancer therapy is chemotherapy.

## Patentansprüche

1. Eine pharmazeutische Zusammensetzung, die aktive Bestandteile umfasst, worin:
- ein erster aktiver Bestandteil Natriumphenylbutyrat in Verbindung mit Valproinsäure ist,
- ein zweiter aktiver Bestandteil Nikotinamid ist, und
- ein dritter aktiver Bestandteil all-trans-Retinsäure (ATRA) ist,
und wahlweise ein oder mehrere pharmazeutisch verträgliche Bindemittel.

2. Die Zusammensetzung gemäß Anspruch 1, die einen aktiven Bestandteil umfasst, fähig, auf DNA-Methyltransferase-(DNMT-)Mechanismen einzuwirken, gewählt aus der Gruppe bestehend aus einem Epigallocatechin und Procain, oder ein verträgliches Salz davon in Verbindung mit Benzoesäure, Kaliummetabisulfit und Dinatriumphosphat.

3. Die Zusammensetzung gemäß Anspruch 1, die Natriumphenylbutyrat in einer Menge zwischen 125 mg und 20 g umfasst, Valproinsäure in einer Menge zwischen 50 mg und 1,6 g, Nikotinamid in einer Menge zwischen 250 mg und 1,5 g und ATRA in einer Menge zwischen 2,5 und 90 mg.

4. Die Zusammensetzung gemäß Anspruch 3, die weiter Procain oder ein verträgliches Salz davon in einer Menge von 50 bis 600mg in Kombination mit Benzoesäure in einer Menge von 6 bis 8mg pro 100 mg Procain umfasst, Kaliummetabisulfit in einer Menge von 3 bis 5 mg pro 100 mg Procain und Dinatriumkaliumphosphat in einer Menge von 0,1 bis 1,5 mg pro 100 mg Procain.

5. Eine Dosierform, insbesondere für die orale Verabreichung, die eine Zusammensetzung gemäß Anspruch 1 umfasst, wobei die Dosierform Folgendes umfasst:
- eine erste magensaftresistente Tablette, die Natriumphenylbutyrat und Valproinsäure einschließt,
- eine zweite Tablette, die Nikotinamid und Procainhydrochlorid in Kombination mit Benzoesäure, Kaliummetabisulfit und Dinatriumkaliumphosphat einschließt, und
- eine einzige weiche Kapsel, die die all-trans-Retinsäure (ATRA) einschließt,
worin wahlweise die erste magensaftresistente Tablette, die zweite magensaftresistente Tablette und die erste einzige weiche Kapsel in eine harte Kapsel integriert werden.

6. Die Dosierform gemäß Anspruch 5, die Folgendes umfasst:
- Natriumphenylbutyrat in einer Menge von 250 mg,
- Valproinsäure in einer Menge von 150 mg,
- Nikotinamid in einer Menge von 250 mg,
- all-trans-Retinsäure (ATRA) in einer Menge von 5 mg.

7. Die Dosierform gemäß einem oder mehreren der Ansprüche 5-6, die Procainhydrochlorid in einer Menge zwischen 20 und 600 mg umfasst und worin jeweils 100 mg Procainhydrochlorid in Kombination mit Benzoesäure in einer Menge zwischen 6 und 8 mg, Kaliummetabisulfit in einer Menge zwischen 3 und 5 mg und Dinatriumkaliumphosphat in einer Menge zwischen 0,1 und 0,5 mg vorliegen.

8. Die Dosierform gemäß Anspruch 7, worin jeweils 100 mg Procainhydrochlorid in Kombination mit Benzoesäure in einer Menge von 7 mg, Kaliummetabisulfit in einer Menge von 4 mg und Dinatriumkaliumphosphat in einer Menge von 1 mg vorliegen.

9. Die Zusammensetzung gemäß einem oder mehreren der Ansprüche 1 bis 4 zur Verwendung in der Behandlung einer Form von Krebs.

10. Die Zusammensetzung zur Verwendung gemäß Anspruch 9, worin die Behandlung durch orale Verabreichung stattfindet.

11. Die Zusammensetzung zur Verwendung gemäß Anspruch 9, worin der Krebs Prostatakrebs ist.

12. Die Zusammensetzung zur Verwendung gemäß Anspruch 9, worin die Zusammensetzung in einer Dosierform gemäß einem oder mehreren der Ansprüche 5-6 vorliegt.

13. Die Zusammensetzung zur Verwendung gemäß Anspruch 12, worin die Zusammensetzung oral verabreicht wird.

14. Die Zusammensetzung zur Verwendung gemäß Anspruch 13, worin die Zusammensetzung mit einer Dosierung von 2 bis 6 der Dosierform pro Tag verabreicht wird.

15. Die Zusammensetzung zur Verwendung gemäß einem der Ansprüche 13 und 14, worin die Zusammensetzung in Kombination mit einer Antikrebstherapie verabreicht wird.

16. Die Zusammensetzung zur Verwendung gemäß Anspruch 15, worin die Antikrebstherapie Chemotherapie ist.

## Revendications

1. Composition pharmaceutique comprenant des ingrédients actifs, où :
- un premier ingrédient actif est le phénylbutyrate de sodium associé à l'acide valproïque ;
- un second ingrédient actif est la nicotinamide ; et
- un troisième ingrédient actif est l'acide tout-trans rétinoique (ATRA),
et comprenant facultativement un ou plusieurs excipients pharmaceutiquement acceptables.

2. Composition selon la revendication 1, comprenant un ingrédient actif capable d'agir sur les mécanismes de la méthyltransférase de l'ADN (DNMT) sélectionné dans le groupe comprenant l'épigallocatéchine et la procaïne ou un sel acceptable de celle-ci, associée à de l'acide benzoïque, du métabisulfite de potassium et du phosphate disodique.

3. Composition selon la revendication 1, comprenant du phénylbutyrate de sodium dans une quantité comprise entre 125 mg et 20 g, de l'acide valproïque dans une quantité comprise entre 50 mg et 1,6 g, de la nicotinamide dans une quantité comprise entre 250 mg et 1,5 g et de l'ATRA dans une quantité comprise entre 2,5 et 90 mg.

4. Composition selon la revendication 3, comprenant en outre de la procaïne ou un sel acceptable de celle-ci dans une quantité comprise entre 50 et 600 mg, associée à de l'acide benzoïque dans une quantité comprise entre 6 et 8 mg pour 100 mg de procaïne, à du métabisulfite de potassium dans une quantité comprise entre 3 et 5 mg pour 100 mg de procaïne et à du potassium disodium phosphate dans une quantité comprise entre 0,1 et 1,5 mg pour 100 mg de procaïne.

5. Forme de dosage, particulièrement destinée à une administration par voie orale, comprenant une composition selon la revendication 1, ladite forme pharmaceutique comprenant :
- un premier comprimé gastrorèsistant contenant du phénylbutyrate de sodium et de l'acide valproïque ;
- un second comprimé contenant de la nicotinamide et du chlorhydrate de procaïne, associés à de l'acide benzoïque, du métabisulfite de potassium et du potassium disodium phosphate : et
- une gélule souple unique contenant l'acide tout-trans rétinoïque (ATRA),
dans laquelle, facultativement, le premier comprimé gastrorésistant, le second comprimé gastrorésistant et la gélule souple unique sont incorporés dans une gélule dure.

6. Forme pharmaceutique selon la revendication 5, comprenant :
- 250 mg de phénylbutyrate de sodium ;
- 150 mg d'acide valproïque ;
- 250 mg de nicotinamide ;
- 5 mg d'acide tout-trans rétinoïque (ATRA).

7. Forme pharmaceutique selon une ou plusieurs des revendications 5 et 6, comprenant du chlorhydrate de procaïne dans une quantité comprise entre 20 et 600 mg et dans laquelle chaque portion de 100 mg de chlorhydrate de procaïne est associée à de l'acide benzoïque dans une quantité comprise entre 6 et 8 mg, à du métabisulfite de potassium dans une quantité comprise entre 3 et 5 mg et à du potassium disodium phosphate dans une quantité comprise entre 0,1 et 0,5 mg.

8. Forme pharmaceutique selon la revendication 7, dans laquelle chaque portion de 100 mg de chlorhydrate de procaïne est associée à 7 mg d'acide benzoïque, à 4 mg de métabisulfite de potassium et à 1 mg de potassium disodium phosphate.

9. Composition selon une ou plusieurs des revendications 1 à 4, destinée à être utilisée dans le traitement d'une forme de cancer.

10. Composition destinée à être utilisée selon la revendication 9, où le traitement est administré par voie orale.

11. Composition destinée à être utilisée selon la revendication 9, où le cancer est un cancer de la prostate.

12. Composition destinée à être utilisée selon la revendication 9, où ladite composition est une forme pharmaceutique selon une ou plusieurs des revendications 5 et 6.

13. Composition destinée à être utilisée selon la revendication 12, où la composition est administrée par voie orale.

14. Composition destinée à être utilisée selon la revendication 13, où la composition est administrée suivant une posologie de 2 à 6 formes pharmaceutiques par jour.

15. Composition destinée à être utilisée selon l'une quelconque des revendications 13 et 14, où ladite composition est administrée en association avec un traitement anti-cancéreux.

16. Composition destinée à être utilisée selon la revendication 15, où le traitement anti-cancéreux est une chimiothérapie.
